Europäisches Patentamt

European Patent Office

Office européen des brevets

(19)

(11) Veröffentlichungsnummer: **0 048 423**

**A2**

(12) # EUROPÄISCHE PATENTANMELDUNG

(21) Anmeldenummer: **81107239.6**

(22) Anmeldetag: **14.09.81**

(51) Int. Cl.³: **A 61 M 5/14**

(30) Priorität: **22.09.80 DE 3035670**

(43) Veröffentlichungstag der Anmeldung:
**31.03.82 Patentblatt 82/13**

(84) Benannte Vertragsstaaten:
**AT CH FR GB IT LI SE**

(71) Anmelder: **SIEMENS AKTIENGESELLSCHAFT Berlin und München**
**Postfach 22 02 61**
**D-8000 München 22(DE)**

(72) Erfinder: **Prestele, Karl, Dipl.-Phys**
**Bismarckstrasse 21d**
**D-8520 Erlangen(DE)**

(72) Erfinder: **Franetzki, Manfred, Dr.-Ing, Dipl.-Phys**
**Schleifweg 7b**
**D-8521 Uttenreuth(DE)**

(72) Erfinder: **Reif, Erich, Dipl.-Ing**
**Obere Gasse 5**
**D-8520 Erlangen(DE)**

(54) **Vorrichtung zur Infusion von Flüssigkeiten in den menschlichen oder tierischen Körper.**

(57) Derartige Vorrichtungen bestehen aus einem Dosiergerät, das am Körper tragbar oder auch vorzugsweise im Körper implantierbar ist, welches eine Fördereinheit zur dosierten Förderung der Flüssigkeit aus einem Vorratsbehälter zur Ausflußöffnung eines Katheters und eine zugehörige Betriebsschaltung aufweist, und aus einem externen Steuer- und Programmiergerät. Die Betriebsschaltung im Dosiergerät und die Steuerschaltung im Steuergerät sind zur betriebsmäßgien Signalübertragung induktiv koppelbar, wozu im Steuergerät wenigstens eine Sendespule und im Dosiergerät wenigstens eine Empfangsspule vorhanden ist. Ziel der Erfindung ist es, bei implantierten Geräten weitestgehende Patientensicherheit zu gewährleisten. Erfindungsgemäß sind Mittel zur Funktionsüberwachung von Betriebsparametern des Dosiergerätes (2) vorhanden, wozu wenigstens das Dosiergerät (2) einen Sender (10, 46, 47) aufweist, dessen Signale in einem Empfänger (52, 53) außerhalb des Patientenkörpers (1) empfangen und gegebenenfalls ausgewertet werden können. Neben der reinen Funktionsüberwachung ist auch eine Quittierung von Signalen sowie weiterhin eine Rückmeldung des Wertes eines oder mehrerer Betriebsparameter möglich.

FIG 4

Croydon Printing Company Ltd

SIEMENS AKTIENGESELLSCHAFT          Unser Zeichen
Berlin und München                  VPA 80 P 5113  E
                          _ 1 _

## Vorrichtung zur Infusion von Flüssigkeiten in den menschlichen oder tierischen Körper

Die Erfindung bezieht sich auf eine Vorrichtung zur Infusion von Flüssigkeiten in den menschlichen oder tierischen Körper, bestehend aus einem Dosiergerät, das am Körper tragbar oder auch vorzugsweise im Körper implantierbar ist, welches eine Fördereinheit zur dosierten Förderung der Flüssigkeit aus einem Vorratsbehälter zur Ausflußöffnung eines Katheters und eine zugehörige Betriebsschaltung aufweist, sowie aus einem externen Steuer- und/oder Programmiergerät, wobei die Betriebsschaltung im Dosiergerät und die Steuerschaltung im Steuergerät zur betriebsmäßigen Signalübertragung induktiv koppelbar sind, wozu im Steuergerät wenigstens eine Sendespule und im Dosiergerät wenigstens eine Empfangsspule vorhanden ist.

Eine solche Vorrichtung soll insbesondere zur Insulininfusion für die Diabetes-Therapie als sog. "künstliches Pankreas" verwendbar sein.

Aus der DE-PS 25 13 467 ist ein Gerät der eingangs genannten Art bekannt, bei dem einem implantierbaren Gerätegehäuse mit Förder- und Dosiereinheit ein externes Steuer- und/oder Programmiergerät zugeordnet ist. Das Steuergerät ist dabei als Programmgeber für ein Schaltventil im Dosiergerät aufgebaut, welcher im einzelnen einen Steuersignalgeber, einen Programmspeicher, einen Programmeingeber für die Eingabe eines Zeit- oder auch Schaltfrequenzprogrammes sowie einen Informationsgeber

Wht 5 Rl / 03.09.1980

zur Anzeige beispielsweise des Programmstandes, der Uhrzeit oder anderer Informationen beinhaltet. Darüber hinaus ist aus der DE-OS 26 51 962 ein implantierfähiges Infusionsgerät vorbekannt, bei dem die Förder- und Dosiereinheit speziell durch eine Rollenpumpe, die durch einen Schrittmotor gesteuert wird, gebildet ist. Dabei ist bereits vorgesehen, den Lauf der Rollenpumpe dadurch zu überwachen, daß jeder Pumpenrolle ein Magnet zugeordnet ist, der bei Pumpenlauf periodisch einen Reed-Kontakt schließt und ein Signal erzeugt. Diese Kontrollsignale dienen in der Betriebsschaltung des implantierten Gerätes im wesentlichen zur Nachsteuerung der Rollenpumpe. Es ist also beim Stand der Technik eine Rückkopplung der Funktion von Förder- und Dosiereinheit auf die Betriebsschaltung im implantierten Gerät vorgesehen.

Aufgabe der Erfindung ist es, Infusionsvorrichtungen des Standes der Technik derart weiterzubilden, daß auch bei im menschlichen Körper implantiertem Dosiergerät jederzeit eine Funktionskontrolle gewährleistet ist. Neben der Überwachung der Funktion des Dosiergerätes soll dabei auch eine Übertragung von Infusionsprogrammen, die in der Betriebsschaltung zugeordneten Speichern abgelegt werden, kontrollierbar sein.

Die Aufgabe wird erfindungsgemäß dadurch gelöst, daß Mittel zur Funktionsüberwachung von Betriebsparametern des Dosiergerätes vorhanden sind, wozu wenigstens das Dosiergerät einen Sender aufweist, dessen Signale in einem Empfänger außerhalb des Patientenkörpers empfangen und gegebenenfalls ausgewertet werden können.

Mit der Erfindung ist eine komplette Funktionsüberwachung der körperinternen und körperexternen Geräteeinheiten möglich. Dabei kann mittels eines separaten Überwachungsgerätes einerseits die rein betriebsmäßige Funktion der Förder- und Dosiereinheit erfaßt werden. Wird als Fördereinheit eine Rollenpumpe mit Schrittmotorantrieb verwendet, so wird bei Ansteuerung des Schrittmotors ein magnetisches Streufeld erzeugt, welches als Signal im separaten Überwachungsgerät empfangen werden kann. Jeder Motorschritt kann dann in ein akustisches und/oder optisches Signal gewandelt werden. Mit einem zusätzlichen Frequenzmesser kann die Schrittmotorfrequenz gemessen und daraus beispielsweise direkt die Infusionsrate bestimmt werden, die an einer Anzeigeeinheit am Überwachungsgerät speziell für die Insulininfusion nach Eichung unmittelbar in Insulineinheiten pro Stunde (IE/h) anzeigbar ist.

Bei erfindungsgemäßen Vorrichtungen ohne Schrittmotorantrieb ist eine zusätzliche Induktionsspule als separater Sender im Dosiergerät vorgesehen.

In Weiterbildung kann mit einer derartigen erfindungsgemäßen Vorrichtung auch der Steuer- bzw. Programmiervorgang des Dosiergerätes überwacht werden. Weist beispielsweise das externe Steuergerät einen Kodierer und die Betriebsschaltung im Dosiergerät einen Dekodierer auf, so daß vom Steuergerät die Steuer- und/oder Programmiersignale kodiert zum implantierten Kodiergerät übertragen und dort vor Weiterverarbeitung dekodiert werden, so kann der Schrittmotor bzw. Sender im implantierten Dosiergerät unmittelbar vom Dekodierer angesteuert werden, so daß nach Empfang eines Steuer- und/oder Programmiersignals ein Ausgangssignal erzeugt wird. Eingangssignal und Ausgangssignal der Betriebsschaltung sind also miteinander zeitlich korreliert, was durch Überprüfung der zeitlichen Koinzidenz der Signale verifizierbar ist. Es ist so also die Möglichkeit der Quittierung von Steuer- und/oder Programmiersignalen gegeben.

In zusätzlicher erfinderischer Weiterbildung kann das Quittiersignal auch zur Rückmeldung eines oder mehrerer geräteinterner Parameter verwendet werden. Während bei einer reinen Quittierung mit einer Koinzidenzschaltung lediglich registriert wird, ob in einem bestimmten Erwartungsintervall nach Umsteuerung bzw. Umprogrammierung im externen Kontrollgerät ein vom Schrittmotor bzw. Sender im implantierten Dosiergerät erzeugtes Signal ankommt, wird in dieser Ausbildung der Erfindung eine definierte Zeitverzögerung des Quittiersignales zur weiteren Informationsübertragung verwendet. Im implantierten Dosiergerät müssen dafür Meßwertgeber zur Erzeugung von den Betriebsparametern entsprechenden elektrischen Signalen und Signal-Zeitwandler, welche als steuerbare Verzögerungsglieder dienen, vorhanden sein.

Spezielle Betriebsparameter zur Rückmeldung können beispielsweise der Innendruck im Dosiergerät, der ein Maß für den Füllungsstand des Vorratsbehälters darstellt, oder auch der Druck im Fördersystem sein. Insbesondere bei letzterem ergibt sich gezielt die Möglichkeit, das Fördersystem auf Strömungshindernisse oder Verstopfungen zu überwachen und bei Erreichen bestimmter Grenzwerte des Druckes entsprechende Gegenmaßnahmen einzuleiten. Daneben kann der rückzumeldende Betriebsparameter beispielsweise auch die seit dem letzten Programmiervorgang oder fortlaufend durch Summation der Steuerimpulse in einem Zähler aufaddierte Insulindosis sein.

Zur Realisierung der Rückmeldung beeinflußt der Meßwert des Signalwandlers für je einen Betriebsparameter über je ein Zeitverzögerungsglied die im Dosiergerät erzeugten Sendesignale. Im externen Steuer- und/oder Programmiergerät werden die Signale des Dosiergerätes in Korrelation zum Steuer- bzw. Programmierimpuls erfaßt und die zeitliche Verzögerung zwischen den Signalen als Information ausgewertet. Die Informationsverarbeitung kann dabei vereinfacht werden, in dem lediglich das Überschreiten von vorgegebenen Grenzwerten erkannt wird. Zur Messung der als Zeitabstände codierten Betriebsparameter können im externen Kontrollgerät analoge oder auch digitale Zeitmeßverfahren angewandt werden. Besonders einfach ist es dabei, wenn der Zeitmesser aus einem einem Taktgeber nachgeschalteten Zähler besteht, wobei Taktgeber oder Zähler vom Steuer- und/oder Programmiersignal gestartet und der Zählerstand beim Eintreffen der jeweiligen Rückmeldesignale ausgegeben wird. Dies kann durch Zwischenspeicherung und/oder Anzeige erfolgen.

Neben der Überwachungs-, Quittierungs- und Rückmeldefunktion ist es mit der Erfindung nun auch möglich, unter Umgehung der Betriebsschaltung mit Speichereinheiten bei an sich programmierbaren Dosiergeräten die Fördereinheit direkt anzusteuern. Da nach jedem Programmiersignal auch ein oder mehrere Motorschritte ausgelöst werden, kann durch entsprechend häufige Wiederholung von Programmiersignalen der Schrittmotor mit beliebiger Frequenz direkt von außen gesteuert werden. Die Programmiersignale werden in diesem Fall zweckmäßig so gewählt, daß die Programmspeicher im Dosiergerät jeweils auf Null gesetzt werden, so daß keine Interferenz von externer Steuerung und Eigensteuerung durch die Betriebsschaltung auftreten kann.

Weitere Einzelheiten und Vorteile der Erfindung ergeben sich aus der nachfolgenden Figurenbeschreibung von Ausführungsbeispielen anhand der Zeichnung in Verbindung mit den weiteren Unteransprüchen.

Es zeigen:

Fig. 1 einen Patienten mit implantiertem Dosiergerät und externem Steuer- und Porgrammiergerät,

Fig. 2 den Aufbau des implantierten Dosiergerätes,

Fig. 3 den Aufbau eines separaten Überwachungsgerätes,

Fig. 4 ein Blockschaltbild der implantierten Betriebsschaltung in Wirkverbindung mit der externen
Schaltung zwecks Quittierung von Steuer- und/
oder Programmiersignalen,

Fig. 5 den Aufbau einer Fig. 4 entsprechenden Schaltung zur Rückmeldung von n Betriebsparametern
des Dosiergerätes und

Fig. 6 den Aufbau einer Fig. 5 entsprechenden Schaltung zur Rückmeldung eines Parameters mit veränderlichem Widerstand als Signalgeber und
eines zugehörigen externen Kontrollgerätes.

In den Figuren sind identische Teile mit den gleichen
Bezugszeichen versehen.

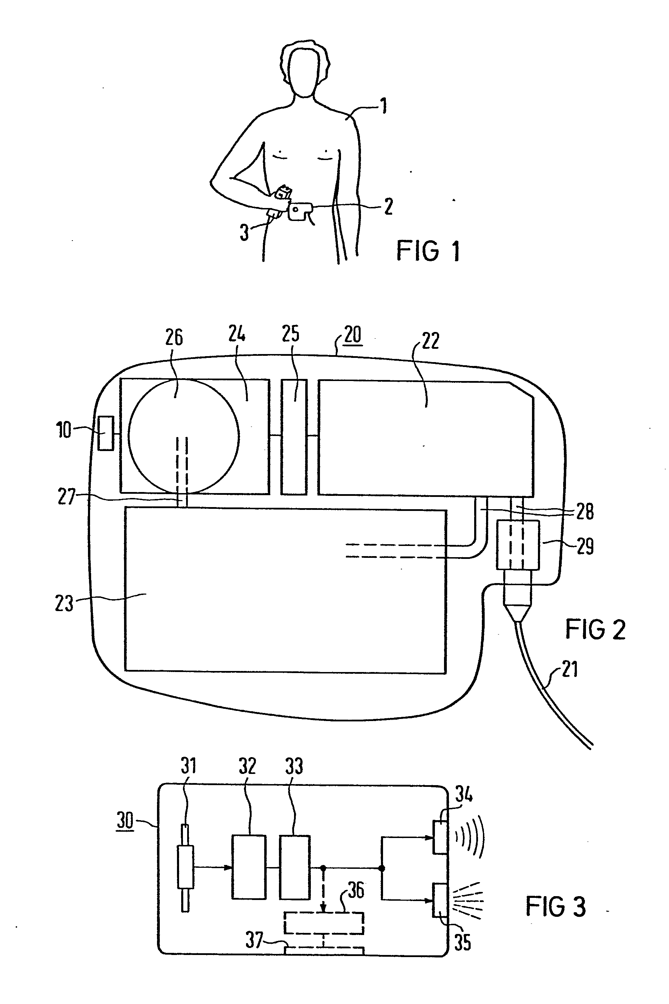

Die Fig. 1 zeigt einen Patienten 1, dem ein Dosiergerät
2 implantiert ist. Das Implantat besteht aus einer
flachen Kapsel mit Flüssigkeitsauslaß, die unter der
Haut im Muskel- bzw. Fettgewebe plaziert ist. Vom
Patienten 1 bzw. vom Arzt ist in Höhe des implantierten
Dosiergerätes 2 extern ein Steuer- bzw. Programmiergerät 3 anlegbar. Durch induktive Signalübertragung
kann derart die Betriebsschaltung im implantierten
Dosiergerät zur Umsteuerung der Infusionsrate bzw. zur
Übernahme eines Infusionsprogrammes aktiviert werden.
Dazu weist die Betriebsschaltung im Dosiergerät 2 entsprechende Speichereinheiten auf, die im einfachsten Fall aus einem ersten Zähler für die Festlegung einer Basalrate und einem zweiten Zähler für die Festlegung einer zeitlich begrenzten Zusatzrate der Infusion bestehen können. Es können jedoch auch Speicher mit größerer Speicherkapazität, beispielsweise sog. RAM's oder sog. FIFO's, verwendet werden. In solche Halbleiterspeicher wird mit einem einzigen Programmiervorgang durch entsprechend kodierte Impulsslaven entweder ein Tagesprogramm oder auch ein Kurzprogramm des Infusionsverlaufes eingelesen. Solche gespeicherten Infusionsprogramme werden mit Hilfe der Betriebsschaltung in entsprechender Zeitkorrelation zyklisch oder einmalig abgearbeitet.

In der Fig. 2 kennzeichnet 20 die Gehäusekapsel des Dosiergerätes 2 nach Fig. 1. Diese ist zweckmäßig in ähnlich flacher Form wie ein Herzschrittmacher ausgebildet und besteht aus körperverträglichem Material, beispielsweise aus Titan. Das Gehäuseinnere ist hermetisch abgeschlossen; ein Ausflußkatheter 21 führt vom Gehäuseauslaß beispielsweise in eine Körpervene. Im Kapselinneren befindet sich eine Fördereinheit 22, mit der aus einem Vorratsbehälter 23 dosiert Infusionsflüssigkeit zur Ausflußöffnung des Katheters 21 gefördert wird. Mit 24 und 25 sind Einheiten angedeutet, die die oben erwähnte Betriebsschaltung mit Speichereinheiten sowie eine Batterie zur elektrischen Versorgung beinhalten. Ein Nachfüllventil 26 hat einen selbstdichtenden Verschluß, durch den beim implantierten Dosiergerät mittels einer Spritze od. dgl. perkutan Flüssigkeit nachgefüllt werden kann. Über eine Verbindungsleitung 27 gelangt die Flüssigkeit vom Nachfüllventil 26 in den Vorratsbehälter 23 und von dort über eine weitere Verbindungsleitung 28 über die Förder- und Dosiereinheit 22 zum Anschluß des Ausflußkatheters 21.

0048423

Mit 29 ist weiterhin ein Drucksignalgeber angedeutet,
der sich zwischen der Fördereinheit 22 und dem Anschluß
des Ausflußkatheters 21 befindet. Als Fördereinheit 22
wird beispielsweise eine Rollenpumpe verwendet. Die
Leitung 28 kann dann unmittelbar den Förderschlauch
der Rollenpumpe bilden, der vom Vorratsbehälter 23
bis zum Anschluß des Katheters 21 führt. Der Drucksignalgeber 29 ist in diesem Fall am endständigen Teil
des Förderschlauches angeordnet. Dessen Funktion wird
weiter unten noch erläutert.

Wie bereits angedeutet, wird die Betriebsschaltung
mit Steuer- und Speichereinheiten innerhalb der Kapsel
20 des Dosiergerätes 2 vom externen Steuer- und Programmiergerät 3 durch induktive Signalübertragung aktiviert. Dazu weist die externe Steuerschaltung einen Sender
zur Erzeugung eines magnetischen Wechselfeldes und die
interne Steuerschaltung einen induktiven Empfänger auf.
Um Fehlübertragungen auszuschließen, werden die Signale
kodiert übertragen. Das externe Steuer- und Programmiergerät 3 beinhaltet dafür einen Kodierer und das interne
Dosiergerät einen Dekodierer. Vom Dekodierer werden
die als Impulssalve empfangenen Signale entsprechend
dem vorgegebenen Code entschlüsselt. Bei betriebsmäßiger Steuerung des Dosiergerätes durch das externe
Gerät aktiviert ein dekodiertes Signal direkt den
Treiber der Fördereinheit. Bei Programmübertragungen
wird dagegen ein ganzes Infusionsprogramm als Impulssalve codiert übertragen, vom Dekodierer dekodiert
und entsprechend in die Speicher des Dosiergerätes
abgelegt. Der Motortreiber wird in diesem Fall entsprechend dem abgelegten Programm aktiviert.

Wird als Fördereinheit 22 eine Rollenpumpe verwendet,
die von einem Schrittmotor angetrieben ist, so wird
bei Ansteuerung des Schrittmotors ein magnetisches Streufeld erzeugt. Dieses Magnetfeld kann etwa bis zu einem gewissen Abstand (ca. 0,15 m) durch eine Induktionsspule mit nachfolgendem Verstärker detektiert und durch optische und/oder akustische Signale angezeigt werden. Durch Empfang dieses magnetischen Streufeldes kann also bereits eine Überwachung des Dosiergerätes 2 realisiert werden.

Alternativ dazu kann in der Kapsel 20 des Dosiergerätes 2 ein spezieller Sender 10 vorhanden sein. Ein solcher Sender kann entweder Einzelimpulse oder Signale mit definierter Trägerfrequenz erzeugen, wobei im letzteren Fall auch der Eingangskreis des externen Empfängers auf die Trägerfrequenz abgestimmt wird. Auf diese Weise läßt sich eine größere Reichweite und/oder Störsicherheit der Übertragung erzielen.

In der Fig. 3 kennzeichnet 30 das Gehäuse eines separaten Überwachungsgerätes. Im Gehäuse 30 befindet sich eine Induktionsspule 31 als Empfangsspule, der eine Verstärker- und Gleichrichtereinheit 32 sowie ein Pulsformer 33 nachgeschaltet sind. Vom Pulsformer 33 werden einerseits eine akustische Anzeigeeinheit 34 und andererseits eine optische Anzeigeeinheit 35 aktiviert. Jedes empfangene Signal, beispielsweise jeder Impuls des geräteinternen Schrittmotors, ist anzeigbar und insbesondere abhörbar. Dabei können bei nicht selektivem Verstärker 32 nicht nur die Signale der implantierten Betriebsschaltung überwacht werden, sondern auch die Signale des externen Steuer- und Programmiergerätes 3. Hinter dem Pulsformer 33 kann weiterhin ein Frequenzmesser 36 geschaltet werden. Vom Frequenzmesser 36 wird die Frequenz der empfangenen Signale, die beispielsweise der Schrittmotorfrequenz entspricht und damit der momentanen Förderrate des Dosiergerätes proportional ist, gemessen. An einer Anzeigeeinrichtung

37 mit digitalem Display ist diese Frequenz anzeigbar. Die Anzeigeeinheit 37 kann direkt in Einheiten der Förderrate, wie beispielsweise Insulineinheiten pro Stunde (IE/h), geeicht sein.

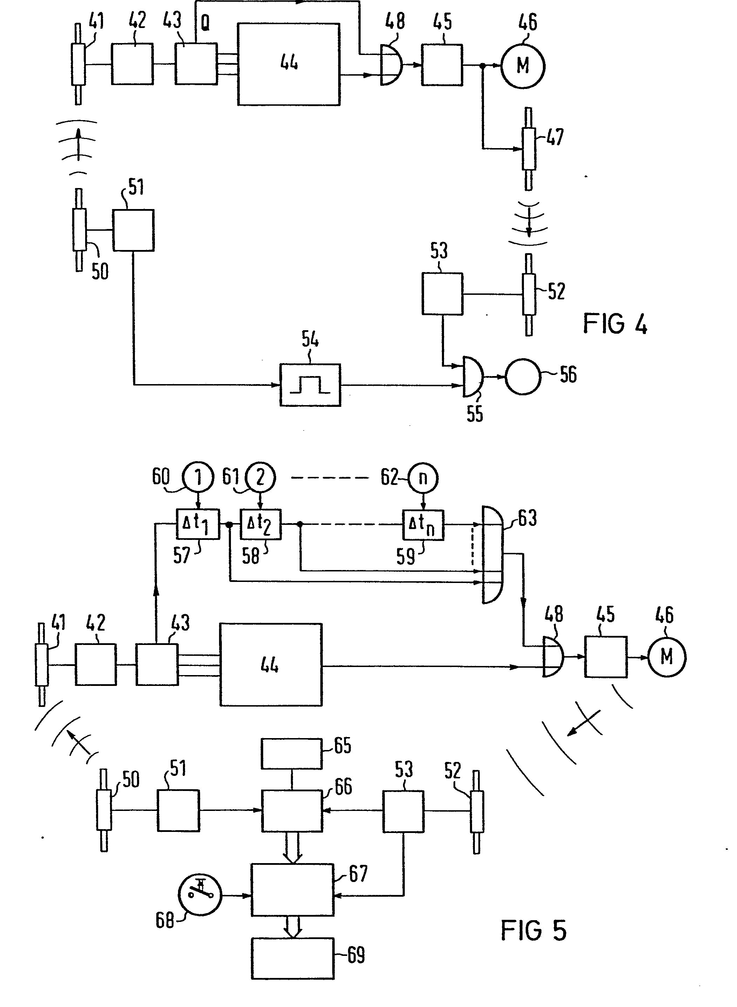

In der Fig. 4 bedeutet 41 eine Empfängerspule im implantierten Dosiergerät 2. Von der Empfängerspule 41 wird über einen Empfangsverstärker 41 ein Dekodierer 43 angesteuert, dem als wesentlicher Teil der Betriebsschaltung des Dosiergerätes 2 eine Speicher- und Steuerschaltung 44 nachgeschaltet ist. Letztere steuert den Motortreiber 45 eines Schrittmotors 46 für die Fördereinheit an. Soweit entspricht dies der vorbekannten Betriebsschaltung des implantierten Dosiergerätes. Vom Dekodierer 43 wird erfindungsgemäß bei jeder Entschlüsselung einer von der Empfängerspule 41 nach vorgegebenem Code empfangenen Impulssalve ein zusätzliches Ausgangssignal erzeugt, das unter Umgehung der Speicher- und Steuerschaltung 44 über ein logisches ODER-Glied 48 direkt den Motortreiber aktiviert.

Bei Dosiergeräten ohne Schrittmotor bzw. zur Erzielung einer größeren Reichweite und/oder Störsicherheit kann fakultativ auch eine separate Induktionsspule 47 direkt oder mit vorgeschaltetem Oszillator zur Erzeugung einer Trägerfrequenz aktiviert werden. Auf diese Weise erhält man ebenfalls magnetische Signale, welche von einem Empfänger, beispielsweise nach Fig. 3, außerhalb des Patientenkörpers empfangen werden können.

Durch obige Schaltung wird also gewährleistet, daß bei jeder Signalübertragung vom externen Steuer- und Programmiergerät 3 zum Dosiergerät 2 ein Ausgangssignal im Dosiergerät 2 erzeugt wird. Dabei kann die Signalübertragung ursächlich der Steuerung bzw. Umsteuerung des Dosiergerätes 2 aber auch einer Übertragung eines ganzen Infusionsprogramm gelten. Ausgangssignale im
Dosiergerät 2 werden aber auch dann erzeugt, wenn der
Motortreiber 45 bei Abarbeitung des in den Speichereinheiten abgelegten Infusionsprogrammes entsprechend vorgegebener Zeitkorrelation angesteuert wird.

Zur Quittierung der Steuer- und Programmiersignale ist
das externe Steuer- und Programmiergerät 2 mit Mitteln
zur Koinzidenzmessung der Signale komplettiert. Die
Steuerschaltung weist üblicherweise eine Sendespule
50 auf, die von einem Sender 51 angesteuert ist. Zusätzlich ist eine Empfangsspule 52 vorhanden, die einen
Empfänger 53 ansteuert. Zum Koinzidenzvergleich triggert
das letzte Signal der Sendesalve ein Monoflop 54 mit vorgegebener Impulszeit. Die Ausgänge des Monoflops 54 und
des Empfängers 53 sind über ein logisches UND-Glied 55,
das eine Anzeigeeinheit 56 ansteuert, verknüpft. Nur
dann, wenn ein Empfangssignal in dem durch das Monoflop 54 vorgegebenen Zeitintervall nach dem Sendesignal
erfaßt wird, erfolgt  an dem Signalgeber 56 eine Anzeige
und damit die Quittierung des Sendesignals. Wird im vorgegebenen Zeitintervall kein Quittiersignal empfangen, so
muß der Programmiervorgang nach Verkleinerung des Abstandes zwischen Programmiergerät 3 und Dosiergerät 2
wiederholt werden.

In der Fig. 5 kennzeichnen die Positionen 41 bis 46, 48
sowie 51 bis 56 die identischen Einheiten der Fig. 4.
In der Umgehungsleitung zur Betriebsschaltung 44 mit
Steuer- und Speichereinheit sind einzelne steuerbare
Verzögerungsglieder 57 bis 59 hintereinandergeschaltet.
Jedes Verzögerungsglied 57 bis 59 wird durch einen der
Meßwertgeber 60 bis 62 angesteuert. Das elektrische Signal
des jeweiligen Meßwertgebers ist durch den Wert eines
physikalischen oder elektrischen Betriebsparameters
bestimmt. Beispielsweise ist ein erster Meßwertgeber ein Druck-/Widerstandswandler, mit dem der Gehäuseinnendruck, der ein Maß für den Füllstand des Vorratsbehälters 22 sein kann, bestimmt wird. Mit einem
zweiten Meßwertgeber kann auch der Druck im Fördersystem selbst erfaßt werden, wodurch Flußbehinderungen
oder auch Verstopfungen in den dünnlumigen Schläuchen,
erkannt werden können. Schließlich kann mit einem
dritten Meßwertgeber auch die seit dem letzten Programmiervorgang oder fortlaufend durch Summation der Steuerimpulse in einem Zähler aufaddierte Infusionsdosis als
Steuergröße für ein Verzögerungsglied verwendet werden.
Weitere Meßwertgeber für andere Geräteparameter sind
denkbar. In der Fig. 5 sind n einzelne Meßwertgeber mit
jeweils einem zugehörigen Verzögerungsglied dargestellt.

Die Ausgänge der Verzögerungsglieder 57 bis 59 sind über
ein n-faches ODER-Glied 63 logisch verknüpft. Dessen
Ausgang führt wiederum über das ODER-Glied 48 und Motortreiber 46 zur Motorspule 47. Mit den Verzögerungsgliedern 57 bis 60 werden also entsprechend den Meßwerten
der zu überwachenden Parameter Impulse bestimmter Länge
$\Delta t_i$ (i = 1 bis n) erzeugt. Jeweils die abfallende Flanke
eines Impulses triggert das nachfolgende Monoflop. Über
die ODER-Glieder 63 und 48 wird gleichzeitig der Motortreiber 45 und die Motorspule 46 aktiviert.

Der Zeitmesser im externen Kontrollgerät ist in diesem
Fall als ein von einem Taktgeber 65 angesteuerter Zähler
66 realisiert. Vom Sender 51 wird jeweils bei Abschluß
einer Programmiersalve der Zähler 66 zurückgesetzt und
anschließend zur Aufzählung der Impulse gestartet. Jeweils bei Detektion eines vom Dosiergerät gesendeten
Rückmeldesignals wird vom Empfänger 53 der Zähler 66 gestoppt und der jeweilige Zählerstand in einem FIFO 67 abgelegt. Anschließend wird der Zähler 66 wieder zurückgesetzt. Zur Zählerstandsübernahme und anschließender Rücksetzung ist eine geeignete Ablaufsteuerung vorhanden.

Bei Übertragung mehrerer Betriebsparameter können also im FIFO 67 nacheinander die Zählstände des Zählers 66 als Information gespeichert werden. Das Auslesen erfolgt dementsprechend nacheinander, was durch Abrufen mit einem handbetätigbaren Taster 68 möglich ist. Die Meßwerte werden auf einer digitalen Anzeigeeinheit 69 angezeigt, die gegebenenfalls für die Betriebsparameter geeicht ist.

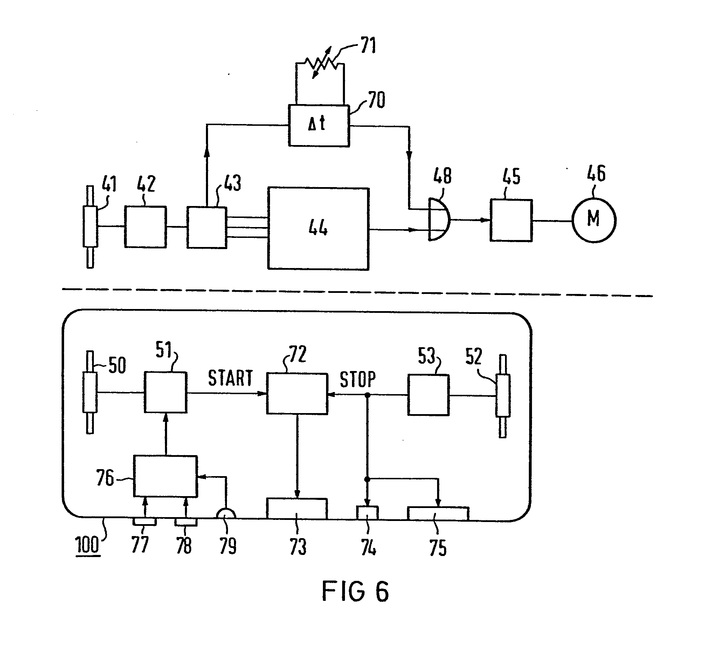

In der Fig. 6 kennzeichnen 41 bis 46 und 48 sowie 50 bis 53 wiederum die oben beschriebenen Einheiten. Vom Dekodierer 43 wird wiederum der Motortreiber 45 unter Umgehung der Speicher- und Steuerschaltung 44 angesteuert. In die Steuerleitung ist speziell ein Monoflop 70 eingeschaltet, das vom Ausgangssignal des Dekodierers 43 gesetzt wird. Zur Einstellung der Verzögerungszeit ist der zeitbestimmende Widerstand des Monoflops 70 als veränderlicher Widerstand eines Druck-/Widerstandswandlers 71 ausgebildet. Proportional zum Wert des Widerstandes wird also über die Steuerleitung der Motortreiber 45 zeitlich verzögert aktiviert und das Rückmeldesignal im definierten Zeitabstand gesendet.

In der externen Steuerschaltung ist dementsprechend ein Zeitmeßglied 72 vorhanden. Das Zeitmeßglied wird vom Sender 51 des externen Steuergerätes mit dem letzten Signal der Sendesalve gestartet und vom Empfänger 53 bei Empfang des Quittiersignals gestoppt. Die gemessene Zeit entspricht dem Meßwert des Druck-/Widerstandswandlers 71 und ist auf einem Display 73 anzeigbar. Weiterhin sind hier Anzeigeeinheiten 74 und 75 vorhanden, mit denen in bekannter Weise jedes im externen Gerät empfangene Signal, d.h. jeder Motorpuls, signalisiert bzw. die Frequenz der Signale gemessen und angezeigt wird. Daneben sind noch die für die Steuerung bzw. Programmierung notwendige Kodierschaltung 76 mit Eingabeeinheiten 77 und 78 sowie ein separater Eingang 79 zur externen Ansteuerung angedeutet. Insgesamt bildet das Steuer- und Programmiergerät mit Empfänger und Schaltungen zur Quittierung der
Steuer- bzw. Programmierimpulse und zur Erfassung eines
rückgemeldeten Betriebsparameters ein komplettes Kontrollgerät 100.

Mit der Schaltungsanordnung nach Fig. 5 und 6 ist also
über die reine Signalquittierung zusätzlich eine Rückmeldung eines Betriebsparameters des implantierten Dosiergerätes möglich. Allerdings ist durch die Zeitmessung im
Kontrollgerät gleichermaßen die Koinzidenzüberprüfung der
Signale und damit die Quittierung des Steuer- und/oder
Programmiervorgangs impliziert. Da alle Signalübertragungsvorgänge im Millisekundenbereich ablaufen, werden
insgesamt bei der Rückmeldung Verzögerungszeiten von
weniger als 100 ms erreicht.

In Fig. 6 soll der Meßwertgeber speziell ein Druck-/
Widerstandswandler sein. Bereits in der Darstellung nach
Fig. 2 wurde ein solcher Drucksignalgeber zur Überwachung
des Druckes im Flüssigkeitsfördersystem mit der Position
29 angegeben. Im einfachsten Fall kann das beispielsweise
ein Druckschalter sein, der bei Überschreiten eines
Grenzwertes anspricht; damit kann bereits eine Störung
im Fördersystem angezeigt werden, womit geeignete Gegenmaßnahmen ergriffen werden können.

Bei den Ausführungsformen der Erfindung nach Fig. 4 bis
6 wurde  der Empfangsspule 41 im Dosiergerät jeweils eine
separate Sendespule zugeordnet. Daneben wurde zur Erweiterung des externen Steuer- und/oder Programmiergerätes
zu einem Kontrollgerät der Sendespule 50 ebenfalls eine
Empfangsspule zugeordnet. Es ist natürlich möglich, jeweils nur eine Spule vorzusehen, die abwechselnd die
Empfangs- bzw. Sendefunktion und umgekehrt übernimmt. Dafür sind dann zusätzlich elektronische oder elektromechanische Umschalter vorzusehen, welche die Spule jeweils vom Empfängereingang auf den Sendeeingang und umgekehrt umschalten.

Bei Beschreibung der dargestellten Ausführungsbeispiele wurde davon ausgegangen, daß in das implantierte Dosiergerät ein gesamtes Infusionsprogramm als kodierte Signalsalve übertragen wird. Mit der erfindungsgemäßen Vorrichtung ist nun aber bei solchen Dosiergeräten, die an sich für eine Programmierung ausgelegt sind, auch eine direkte Steuerung des Dosiergerätes unter Umgehung des abgelegten Programmes möglich. Dafür werden mit einem Frequenzgeber über   den externen Eingang 79 am Kontrollgerät 100 Signalfolgen mit der gewünschten Frequenz kodiert übertragen, die den Motortreiber direkt vom Dekodierer her mit der vorgegebenen Frequenz ansteuern. Auf diese Weise kann z.B. mit Hilfe eines extrakorporalen Glukosesensors und eines geeigneten Regelalgorithmus' für eine bestimmte Zeit eine glukosegeregelte Insulinfusion erzwungen werden, was insbesondere in kritischen Patientensituationen oder zur Ermittlung eines optimalen Infusionsprogramms sinnvoll sein kann.

6 Figuren
28 Patentansprüche

Patentansprüche

1. Vorrichtung zur Infusion von Flüssigkeiten in den menschlichen oder tierischen Körper, bestehend aus einem Dosiergerät, das am Körper tragbar oder auch vorzugsweise im Körper implantierbar ist, welches eine Fördereinheit zur dosierten Förderung der Flüssigkeit aus einem Vorratsbehälter zur Ausflußöffnung eines Katheters und eine zugehörige Betriebsschaltung aufweist, sowie aus einem externen Steuer- und/oder Programmiergerät, wobei die Betriebsschaltung im Dosiergerät und die Steuerschaltung im Steuergerät zur betriebsmäßigen Signalübertragung induktiv koppelbar sind, wozu im Steuergerät wenigstens eine Sendespule und im Dosiergerät wenigstens eine Empfangsspule vorhanden ist, d a d u r c h   g e - k e n n z e i c h n e t ,   daß Mittel zur Funktionsüberwachung von Betriebsparametern des Dosiergerätes (2) vorhanden sind, wozu wenigstens das Dosiergerät (2) einen Sender (10) aufweist, dessen Signale in einem Empfänger (31-33, 52-53) außerhalb des Patientenkörpers (1) empfangen und gegebenenfalls ausgewertet werden können.

2. Vorrichtung nach Anspruch 1, wobei die Fördereinheit durch eine Rollenpumpe mit Schrittmotorantrieb gebildet ist, d a d u r c h   g e k e n n z e i c h n e t , daß der Sender (5) durch die Spule (46) des Schrittmotors gebildet ist.

3. Vorrichtung nach Anspruch 1, d a d u r c h   g e - k e n n z e i c h n e t ,   daß der Sender im Dosiergerät (2) durch eine separate Spule (47) gebildet ist, die gegebenenfalls mit der Empfangsspule (41) identisch ist.

4. Vorrichtung nach Anspruch 1 bis 3, d a d u r c h   g e k e n n z e i c h n e t , daß der Sendespule (47) ein Oszillator zur Erzeugung einer Trägerfrequenz vorgeschaltet ist.

5. Vorrichtung nach Anspruch 1 bis 3, d a d u r c h g e k e n n z e i c h n e t , daß ein separates Über- wachungsgerät (30) vorhanden ist, das wenigstens einen Empfänger (31-33) sowie Anzeigemittel (34, 35) für die Signale des Senders (10, 46, 47) aufweist.

6. Vorrichtung nach Anspruch 5, d a d u r c h g e - k e n n z e i c h n e t , daß der Empfänger aus einer Spule (31) mit nachfolgender Verstärker- und Gleichrichterschaltung (32) sowie Impulsformstufe (33) gebildet ist.

7. Vorrichtung nach Anspruch 5, d a d u r c h g e - k e n n z e i c h n e t , däß der Impulsformstufe optische und/oder akustische Anzeigeeinheiten (34, 35) nachgeschaltet sind.

8. Vorrichtung nach Anspruch 6, d a d u r c h g e - k e n n z e i c h n e t , daß der Impulsformstufe (33) ein Frequenzmesser (36) sowie eine Anzeigeeinheit (37) zur Anzeige der Frequenz der Signale nachgeschaltet sind.

9. Vorrichtung nach Anspruch 1, d a d u r c h g e - k e n n z e i c h n e t , daß der Empfänger (52, 53) mit dem Steuer- und Programmiergerät (3) kombiniert wird und dieses zu einem Kontrollgerät (100) erweitert.

10. Vorrichtung nach Anspruch 9, wobei vom Steuer- und Programmiergerät Steuer- und/oder Programmiersignale kodiert zum Dosiergerät übertragen werden, wozu das Steuer- und Programmiergerät einen Kodierer und die Betriebsschaltung im Dosiergerät einen Dekodierer auf- weist, d a d u r c h g e k e n n z e i c h n e t , daß der Sender (46, 47) vom Dekodierer (43) im Dosiergerät (2) angesteuert wird und ein Quittiersignal nach Empfang eines Steuer- und/oder Programmiersignals er- zeugt.

11. Vorrichtung nach Anspruch 10, d a d u r c h   g e - k e n n z e i c h n e t ,   daß der Dekodierer (43) einen separaten Ausgang (Q) zur Umgehung der Betriebsschaltung (44) mit Speichereinheiten aufweist, welcher bei Beendigung eines Dekodiervorganges ein Signal zur Aktivierung des Senders (47) bzw. Schrittmotors (46) abgibt.

12. Vorrichtung nach Anspruch 10, d a d u r c h   g e k e n n z e i c h n e t ,   daß das vom Empfänger (52, 53) des externen Kontrollgerätes empfangene Quittiersignal in einer logischen Schaltung (51, 53-55) auf Koinzidenz mit dem Steuer- und/oder Programmiersignalen geprüft wird.

13. Vorrichtung nach Anspruch 12, d a d u r c h   g e k e n n z e i c h n e t ,   daß zur Erzeugung eines Koinzidenzintervalles ein Monoflop (54) vom Sender (51) des Kontrollgerätes angesteuert wird.

14. Vorrichtung nach Anspruch 12 und 13, d a - d u r c h   g e k e n n z e i c h n e t ,   daß bei Eintreffen eines Quittiersignals während des Koinzidenzintervalles an einer Anzeigeinheit (56) ein akustisches oder optisches Signal ausgelöst wird.

15. Vorrichtung nach Anspruch 8, d a d u r c h   g e k e n n z e i c h n e t ,   daß im Dosiergerät (2) das Quittiersignal in einem definierten zeitlichen Abstand vom empfangenen Steuer- und/oder Programmiersignal erzeugt wird.

16. Vorrichtung nach Anspruch 8 und 15, d a d u r c h   g e k e n n z e i c h n e t ,   daß der zeitliche Abstand des Quittiersignals vom Steuer- und/oder Pro-

0048423

- 19 -     VPA 80 P 5113  E

grammiersignal durch den Wert eines physikalischen bzw.
elektrischen Parameters im implantierten Dosiergerät
(2) bestimmt ist.

17. Vorrichtung nach Anspruch 8 und 15,  d a d u r c h
g e k e n n z e i c h n e t ,  daß im Dosiergerät (2)
mehrere Signale nach einem Steuer- und/oder Programmiersignal erzeugt werden, die nacheinander in Abhängigkeit
von je einem Parameter den Sender (10, 46, 47) des
Dosiergerätes (2) aktivieren, wobei der Abstand zwischen den Signalen dem Wert jeweils eines Parameters
entspricht.

18. Vorrichtung nach Anspruch 16,  d a d u r c h
g e k e n n z e i c h n e t ,  daß im implantierten
Dosiergerät (2) wenigstens ein steuerbares Zeitverzögerungsglied (57-59) vorhanden ist, das von einem
Meßwertgeber (60-62) für den zu überwachenden Parameter
angesteuert ist.

19. Vorrichtung nach Anspruch 18,  d a d u r c h
g e k e n n z e i c h n e t ,  daß mehrere hintereinandergeschaltete und durch verschiedene Meßwertgeber (60-62) angesteuerte Zeitverzögerungsglieder (57-
59) vorhanden sind, wobei das erste Zeitverzögerungsglied (57) durch das Steuer- und/oder Programmiersignal getriggert wird und jeweils bei Triggerung des
nächstfolgenden Verzögerungsgliedes (58, 59) ein Sendeimpuls im Dosiergerät (2) erzeugt wird.

20. Vorrichtung nach Anspruch 16, 17, 18 oder 19,  d a -
d u r c h  g e k e n n z e i c h n e t ,  daß der
Parameter der Innendruck des Dosiergerätes (2) ist.

21. Vorrichtung nach Anspruch 16, 17, 18 oder 19,  d a -
d u r c h  g e k e n n z e i c h n e t ,  daß der Para-

0048423

- 20 -    VPA 80 P 5113 E

meter der Druck im Fördersystem (22, 28) des Dosiergerätes (2) ist.

22. Vorrichtung nach Anspruch 16, 17, 18 oder 19, d a d u r c h  g e k e n n z e i c h n e t , daß der Parameter die seit dem letzten Programmiervorgang oder fortlaufend durch Summation der Steuerimpulse in einem Zähler aufaddierte Infusionsdosis ist.

23. Vorrichtung nach Anspruch 20 oder 21, d a d u r c h  g e k e n n z e i c h n e t , daß der Meßwertgeber einen Druck-/Widerstandswandler (71) darstellt.

24. Vorrichtung nach Anspruch 17, d a d u r c h  g e k e n n z e i c h n e t , daß das Zeitverzögerungsglied ein Monoflop (70) ist, dessen Pulszeit durch den Widerstandswert des Druck-/Widerstandswandlers (71) bestimmt wird.

25. Vorrichtung nach Anspruch 16, d a d u r c h  g e k e n n z e i c h n e t , daß im Kontrollgerät (100) ein Zeitmesser (72) mit Anzeigeeinheit (73) zur Erfassung der Zeitabstände der Rückmeldesignale vom Steuer- und/oder Programmiersignal vorhanden ist.

26. Vorrichtung nach Anspruch 25, d a d u r c h  g e k e n n z e i c h n e t , daß der Zeitmesser aus einem einem Taktgeber (65) nachgeschalteten Zähler (66) besteht, wobei Taktgeber (65) oder Zähler (66) vom Steuer- und/oder Programmiersignal des Senders (51) im Kontrollgerät gestartet und beim Eintreffen der Rückmeldesignale im Empfänger (53) der jeweilige Zählerstand ausgegeben wird.

27. Vorrichtung nach Anspruch 26, d a d u r c h  g e k e n n z e i c h n e t , daß im Kontrollgerät

(100) der Zeitmesser (65, 66) den Zeitabstand der vom Dosiergerät (2) gesendeten Signale nacheinander ausmißt, wobei dem Zeitmesser (65, 66) Mittel (67-69) zur Speicherung und/oder Anzeige der Meßwerte zugeordnet sind.

28. Vorrichtung nach Anspruch 27, d a d u r c h g e k e n n z e i c h n e t , daß zum Zwischenspeichern der Zeitabstände ein FIFO (67) vorhanden ist, in das die Meßwerte nacheinander eingelesen werden und aus dem sie nacheinander über ein Taster (68) abrufbar sind.

FIG 1

FIG 2

FIG 3

FIG 4

FIG 5

0048423

FIG 6